# EUROPEAN PATENT SPECIFICATION

(11) **EP 1 396 268 B1**
(45) Date of publication and mention of the grant of the patent: **07.06.2006**
(21) Application number: 03255566.6
(22) Date of filing: 05.09.2003
(51) Int. Cl.: A61K 31/675, A61K 31/185, A61K 31/724, A61P 35/00

(54) **Stable oxazaphosphorine-2-mercaptoethanesulphonate formulations**
Stabile Oxazaphosphorin-2-Mercaptoethansulphonat Formulierungen
Formulations stables d'oxazaphosphorine-2-mercaptoethanesulfonate

(30) Priority: 05.09.2002 IN MU08092002
(43) Date of publication of application: 10.03.2004
(73) Proprietor: Bharat Serums & Vaccines Ltd., Wagle Estate, Thane 400 604 (IN)
(72) Inventor: Daftary, Gautam Vinod, Wagle Estate, 400 604 Thane (IN); Pai, Srikanth Annappa, Wagle Estate, 400 604 Thane (IN); Rivankar, Sangeeta Hanurmesh, Wagle Estate, 400 604 Thane (IN); Praveen, Kumar Subbappa, Wagle Estate, 400 604 Thane (IN)
(74) Representative: Burford, Anthony Frederick

(56) References cited:
- EP-A- 0 895 783
- WO-A-91/04026
- WO-A-96/30024
- WO-A-03/002101
- US-A- 4 959 215

## Description

This invention relates to a process for preparation of low toxicity, stable aqueous ready-to-use oxazaphosphorine-containing compositions comprising an oxazaphosphorine antineoplastic, a 2-mercaptoethanesulphonate and an etherified β-cyclodextrin. It has particular, but not exclusive, application to the preparation of compositions containing Ifosfamide, Mesna and 2-hydroxypropyl-β-cyclodextrin (referred to hereinafter as "HPBCD") suitable for parenteral administration in human beings and mammals. The invention is more particularly related to a process for preparation of clear aqueous low toxicity compositions of Ifosfamide comprising Ifosfamide, Mesna, HPBCD that are stable over a period of time thereby making them suitable for ready clinical use.

Two main groups of drugs used in the treatment of malignant disease are alkylating agents and the antimetabolites. Ifosfamide is an oxazaphosphorine and one of the widely used antineoplastic drugs belonging to the alkylating agents group. It is given intravenously either by injection as a solution diluted to less than 4% or by infusion and is used in the treatment of a variety of solid tumours including those of the cervix, endometrium, lung, ovary, testes and thymus as well as in sarcoma and in the treatment of Burkitts lymphoma.

Ifosfamide is the Approved Name for 3-(2-chloroethyl)-2-[(2-chloroethyl)amino]-tetrahydro-2H- 1,3,2-oxazaphosphorin-2-oxide and is represented by the formula: 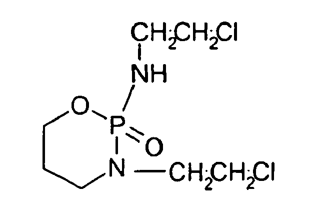 It is a white hygroscopic crystalline powder having a low melting point of 40°C. It also begins to sinter below its melting point. These characteristics of Ifosfamide make it difficult for sterile filling of the dry powder as both temperature and humidity are required to be accurately controlled. Further, as Ifosfamide powder is filled aseptically into sterile containers, maximum precautions are required to maintain sterility of the product.

Even though Ifosfamide powder is freely soluble in water, the solubility decreases on storage. Ifosfamide has been reported to undergo a reversible chemical rearrangement in aqueous solution, which is sensitive to changes in pH. The ratio of these compounds to one another in biological fluids have a bearing on the toxicity and efficacy of Ifosfamide.

Similar problems are encountered with other oxazaphosphorine antineoplastic, e.g. Cyclophosphamide, which is the Approved Name for 2-[bis(2-chloroethyl)amino]-tetrahydro-2H-1,3,2-oxazophosphorine 2-oxide, represented by the formula:

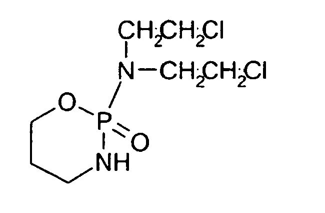

Oxazaphosphorine antineoplastics are toxic to the urinary tract and may involve the kidneys as well as the bladder. Hence it is recommended that they are administered in association with 2-mercaptoethanesulphonates, especially Mesna. Mesna is the Approved Name for sodium 2-mercaptoethanesulphonate and is represented by the formula:

HSCH₂CH₂SO₃ Na⁺.

It is used for the prophylaxis of urothelial toxicity in patients being treated with Ifosfamide or Cyclophosphamide. In the kidney Mesna disulfide, the inactive metabolite of Mesna is reduced to free Mesna, which has thiol groups that react with the metabolites of Ifosfamide, and Cyclophosphamide, including acroleil, considered to be responsible for the toxic effects on the bladder.

The intravenous daily dose of Mesna is calculated to equal 60% of the total daily dose of Ifosfamide and is administered as 3 bolus doses given 15 minutes before and 4 and 8 hours after administration of each dose of Ifosfamide when the Ifosfamide dose is less than 2.5g/day administered as a short infusion. For use with continuous infusion of Ifosfamide, Mesna may be administered as a bolus dose equal to 20% of the total Ifosfamide dose followed by a continuous infusion of Mesna equal to 40% of the Ifosfamide dose, continuing for 12 to 24 hours after completion of the Ifosfamide infusion.

Mesna has also been administered as a continuous infusion at a dose equal to 60% of Ifosfamide dose. No clinical data is available to justify Mesna doses greater than 60% w/w of Ifosfamide for standard doses of Ifosfamide. With high doses in excess of 2.5g/day of Ifosfamide, continuous and prolonged Mesna dosage regimen is necessary for maximum protection against urotoxicity.

The disadvantages with the existing commercially available product in powder form is that
1. more than one vial is required to be reconstituted and then diluted to the required concentration as the standard dosage is more than 1g daily,
2. in high dosage Ifosfamide therapy as high as eight vials of 1g are required to be reconstituted and diluted to the required concentration.
3. as Mesna is required to be administered along with Ifosfamide, Ifosfamide solution after reconstitution is required to be mixed with Mesna.

US-A-4959215 discloses a stable Ifosfamide-Mesna lyophilizate comprising Ifosfamide, 0.05 to 1.0 parts by weight of Mesna and 0.1 to 17 parts by weight of a hexitol prepared by freeze drying an aqueous or aqueous-ethanolic solution of Ifosfamide, Mesna and the hexitol, preferably mannitol. There is no reference to the presence of any cyclodextrin. The lyophilizate is stable physically showing no discolouration. The speed of dissolution is also claimed to markedly higher compared to the dry filled Ifosfamide.

US-A-4952575 discloses a composition comprising 10 to 70 %w/v of an oxazaphosphorine of the formula: 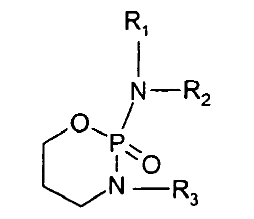 in which at least two of R₁, R₂ and R₃ independently are 2-chloroethyl or 2-methanesulfonyloxyethyl and any remaining R radical is selected from hydrogen, a, methyl and ethyl,
dissolved in 80 to 100 %v/v of ethanol. Even though the degradation has been shown to be minimal for Ifosfamide, use of solvents in such a high concentration leads to other problems such as volatility, handling during manufacturing, miscibility with blood. As ethanol is pharmacologically active, this may also affect the person on administration of alcoholic solution of Ifosfamide.

WO-A-9918973 discloses stable ready-to-use liquid compositions of at least one oxazaphosphorine of the formula: 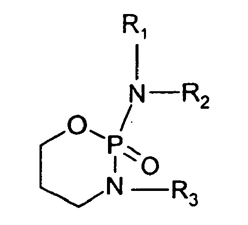 in which R₁, R₂ and R₃ independently are methyl, ethyl, 2-chloroethyl, 2-methanesulfonyloxyethyl or, except for R₃, hydrogen, and at least two of R₁, R₂ and R₃ are 2-chloroethyl and/or 2-methanesulfonyloxyethyl,
comprising a physiologically well-tolerated compound which forms chloride ions in aqueous solution. It is independently stated that the composition can include cyclodextrins, preferably α-cyclodextrins, or their ethoxylated derivatives as tonicity adjustment agents and Mesna but there is no exemplification of any composition containing both a cyclodextrin and Mesna. The product has been shown to be stable at refrigerated temperatures but the stability data provided does not show satisfactory stability at elevated temperatures.

US-A-4879286 discloses a storage-stable liquid oncolytic formulation of Cyclophosphamide formulated as a ready-to-dilute solution in a carrier comprising 50 to 100 % organic polyol selected from propylene glycol, polyethylene glycol and glycerol and 0 to 50 % water. The formulation may be used in combination with alcohols, such as 10 to 30% of ethanol (based on total weight of the formulation). The ready-to-dilute solutions have been shown to be stable under refrigerated conditions but the stability data at elevated temperatures are not sufficient to prove that the product is stable.

US-A-6407079 discloses that the water-solubility and stability of sparingly water-soluble or water-instable drugs are improved by the formation of inclusion compounds with partially etherified β-cyclodextrins of the formula:

(β-CD)OR

in which R are hydroxyalkyl groups with optionally some being alkyl groups, and having a water-solubility of more than 1.8 g in 100 ml water. Preferably, R are selected from hydroxyethyl, hydroxypropyl or dihydroxypropyl groups. There is no mention of either Ifosfamide or Mesna.

US-A-4727064 discloses that lipophilic drugs can be stabilized by solubilizing the drug into an intrinsically amorphous mixture of a water-soluble cyclodextrin derivative to form a solubilized cyclodextrin/drug complex and, optionally, freeze-drying or evaporating the resultant solubilized complex to provide a solid cyclodextrin/drug complex in powder form. The exemplified mixtures of cyclodextrin derivatives are obtained by non-selectively alkylating α-, β-, or γ-cyclodextrin using, for example, propylene oxide, glycidol, iodoacetamide, chloroacetate or 2-diethylaminoethylchloride. The cyclodextrins can be substituted by hydroxyalkyl carboxamide, diethylaminoethyl, carboxymethyl or carboxyamidomethyl and exemplified cyclodextrins include hydroxypropyl-β-cyclodexrin. There is no mention of either Ifosfamide or Mesna.

WO-A-0139749 discloses fast dissolving pharmaceutical compositions in solid dosage form with prolonged sweet taste comprising (a) at least one drug, (b) at least one water soluble sugar, (c) at least one non-sugar sweetener in normal fast release form and (d) at least one non-sugar sweetener in a mucoadhesive slow release form. Exemplified drugs include Ifosfamide and Mesna and exemplified mucoadhesive agents include cyclodextrins. There is no exemplification of any composition containing two or more of Ifosfamide, Mesna and a cyclodextrin.

As Mesna is required to be given at different intervals concurrently during administration of Ifosfamide, in one aspect of the invention Ifosfamide and Mesna are combined in the same composition to avoid the inconvenience of administering Mesna separately. In another aspect of the invention Ifosfamide and Mesna are combined with HPBCD to give a stable composition so that the product is readily marketable and is convenient to use without the step of reconstitution and less handling. Surprisingly, the process of invention in which Ifosfamide, Mesna and HPBCD are combined has produced a composition having low toxicity also.

The main objective of this invention is thus to develop a process for preparing low toxicity, stable compositions of Ifosfamide comprising Ifosfamide, Mesna, HPBCD, with or without conventional parenteral additives, overcoming all the disadvantages of prior arts and make the composition suitable for parenteral administration in human beings and mammals.

In its broadest aspect, the invention provides a process for preparation of a low toxicity, stable oxazaphosphorine-containing composition comprising an oxazaphosphorine antineoplastic, a 2-mercaptoethanesulphonate and an etherified β-cyclodextrin; the process comprising adding the 2-mercaptoethanesulphonate to an aqueous solution of the oxazaphosphorine antineoplastic and etherified β-cyclodextrin.

In one embodiment, the oxazaphosphorine antineoplastic is added to an aqueous solution of the etherified β-cyclodextrin; the 2-mercaptoethanesulphonate is added to an aqueous solution optionally containing an etherified β-cyclodextrin; and both the resultant aqueous solutions are mixed together and, optionally, the volume made up with water.

In another embodiment, the oxazaphosphorine antineoplastic is added to an aqueous solution of the etherified β-cyclodextrin and subsequently the 2-mercaptoethanesulphonate is directly (i.e. without dissolution) added to the oxazaphosphorine-containing solution.

Preferably, the oxazaphosphorine antineoplastic is of the formula: 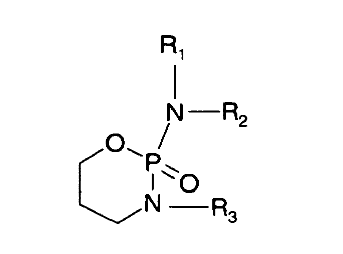 in which at least two of R₁, R₂ and R₃ independently are 2-chloroethyl or 2-methanesulfonyloxyethyl and any remaining R radical is selected from hydrogen, methyl and ethyl. More preferably, the oxazaphosphorine antineoplastic is Cyclophosphamide (R₁ = R₂ = chloroethyl & R₃ = hydrogen) or, especially, Ifosfamide (R₁ = R₃ = chloroethyl & R₂ = hydrogen).

Preferably, the 2-mercaptoethanesulphonate is sodium 2-mercaptoethanesulphonate (i. e. Mesna).

The etherified β-cyclodextrin preferably has at least some of the hydroxy groups etherified with hydroxyalkyl groups and optionally others etherified with alkyl groups and a water-solubility of more than 1.8 g/100 ml water. Preferably the hydroxyalkyl groups are hydroxyethyl, dihydroxypropyl or, especially, hydroxypropyl groups and the alkyl groups, if present, are methyl or ethyl groups; The molar substitution (MS) by hydroxyalkyl groups (calculated as moles of alkylating alkylene oxide per anhydroglucose unit) suitably is 0.05 to 10, preferably 0.2 to 2, and especially 0.25 to 1. The degree of substitution (DS) per glucose unit by alkyl groups, if present, suitably is 0.05 to 2.0, preferably 0.2 to 1.5, and especially 0.5 to 1.2.

Presently, it is especially preferred that the etherified β-cyclodextrin is a hydroxypropyl β-cyclodextrin having a molar substitution per anhydro glucose unit of 0.3 to 0.9, especially 0.5 to 0.8. Such hydroxypropyl β-cyclodextrins are available from Wacker Chemie under the trade mark Cavasol.

The oxazaphosphorine antineoplastic content (calculated as Ifosfamide equivalent) of the composition usually is from 1 mg/ml to 1,000 mg/ml, preferably from 25 mg/ml to 750 mg/ml and especially either 50 mg/ml or 500 mg/ml. Other suitable ranges are from 1 mg/ml to 200 mg/ml; from 10 mg/ml to 100 mg/ml; and from 40 mg/ml to 50 mg/ml.

The ratio of oxazaphosphorine antineoplastic (calculated as Ifosfamide equivalent) to 2-mercaptoethanesulphonate (calculated as Mesna equivalent) usually is in the range of 20 : 1 to 1 : 2 on a weight basis, preferably in the range of 10 : 1 to 1 : 1, especially in the range of 10 : 1 to 1 : 2.

The content of etherified β-cyclodextrin (calculated as β-hydroxypropyl equivalent) in the composition usually is 1% to 60% w/v, preferably 2.5% to 40% w/v, more preferably 10% to 20% w/v, especially 5% to 20% w/v.

Conventional parenteral additives may be present in the aqueous solution to which the oxazaphosphorine antineoplastic is added and/or in any separate aqueous solution to which the 2-mercaptoethanesulphonate is added. Such additives can be, for example buffers, isotonic diluents, anticrystallising agents, sequestering agents, or antioxidants as commonly used in aqueous parenteral compositions, provided that they do not significantly reduce the stability of the product composition. Usually, conventional parenteral additives when added in the usual recommended range do not affect the clarity and stability of the composition adversely.

Buffers are selected from pharmaceutically acceptable buffer systems such as, for example, phosphate buffer, citrate buffer, glycine buffer containing any of the commonly used compounds or a mixture of compounds selected from citric acid, sodium citrate, potassium citrate, glycine, phosphoric acid, sodium phosphate, disodium hydrogen phosphate, sodium dihydrogen phosphate, potassium phosphate, dipotassium hydrogen phosphate, potassium dihydrogen phosphate, sodium hydroxide, potassium hydroxide, hydrochloric acid. Preferably the buffer used is a mixture of sodium dihydrogen phosphate and disodium hydrogen phosphate.

The oxazaphosphorine antineoplastic and 2-mercaptoethanesulphonate are added to the aqueous solution(s) with intimate stirring and the resultant mixture usually sterilized by filtering, especially through a pathogen proof filter. Preferably, the mixture is filtered through 2µ and 0.2µ filters successively or just through a 0.2µ filter.

Usually, the filtrate will be aseptically filled into sterile containers such as vials, ampoules, plastic containers followed by nitrogen purging and sealing the filled containers.

The invention will now be illustrated by way of Examples.

Ifosfamide used in these Examples was of parenteral grade complying with US Pharmacopoeial specifications. Hydroxypropyl-β-cyclodextrin (HPBCD) used was Cavesol^{™} W7 HP Pharma manufactured by Wacker Chemie and, reportedly, having molar substitution of 0.6 to 0.8. Equipment used was of conventional nature; the entire processing was done in an area with a controlled environment. Nitrogen cover was provided while processing the batch.

### Example I:

| | | |
|---|---|---|
| 1. | Ifosfamide | 10g |
| 2. | Mesna | 2g |
| 3. | HPBCD | 40g |
| 4. | Disodium hydrogen phosphate | 0.1g |
| 5. | Sodium dihydrogen phosphate | 0.06g |
| 6. | Water | q.s. to 200 ml |

Weighed quantities of disodium hydrogen phosphate and sodium dihydrogen phosphate were dissolved in 160 ml of water and a weighed quantity of HPBCD was added and dissolved slowly under stirring. The resultant HPBCD solution was divided into two equal parts.

A weighed quantity of Ifosfamide was gradually added under stirring to one part of buffered HPBCD solution and mixed for 3 hours.

A weighed quantity of Mesna was gradually added under stirring to the remaining part of buffered HPBCD solution.

Both the above Ifosfamide and Mesna solution were mixed together and the volume was made up to 200 ml with water. The product was filtered through a 0.2µ filter and filled aseptically in sterile glass vials. The glass vials were closed under aseptic conditions with sterile Teflon^{™} coated rubber bungs and sealed using flip off seals.

The composition obtained in this Example was analysed for Ifosfamide content and Mesna content by high pressure liquid chromatography (HPLC) and was found to contain 52.92 mg/ml of Ifosfamide and 10.2 mg/ml of Mesna. The composition had a pH of 6.86.

### Example II:

The composition obtained in Example I was subjected to acute toxicity studies in mice. A conventional formulation , Holoxan^{TM} supplied by German Remedies Limited was reconstituted as directed by the manufacturer and was used as a control after mixing with Mesna (equivalent to 20% of Ifosfamide content). Both the drug solutions were suitably diluted with 5% Dextrose Injection and administered intravenously. Ifosfamide in the doses of 500 mg/kg, 700 mg/kg and 900 mg/kg body weight was administered in three different groups of animals, each group consisting of eight animals.

The animals were kept under observation for 14 days and mortality recorded at the end of 3 days and 7 days.

It was observed that the LD₅₀ dose was higher for composition of Example I in comparison with the Conventional formulation.

| **Composition of Example I** | | | **Conventional formulation** | | |
|---|---|---|---|---|---|
| **Mortality (%)** | | | **Mortality (%)** | | |
| **Dose (mg)** | **3 Days** | **7 Days** | **Dose (mg)** | **3 Days** | **7 Days** |
| 500 | 0 | 0 | 500 | 50 | 75 |
| 700 | 0 | 50 | 700 | 100 | 100 |
| 900 | 75 | 100 | 900 | 100 | 100 |
| **LD**_{**50**} | **700 - 900** | **700** | **LD**_{**50**} | **500** | **<500** |

The above data clearly indicates that composition of Example I is less toxic compared to the Conventional formulation.

### Example III:

| | | |
|---|---|---|
| 1. | Ifosfamide | 10g |
| 2. | Mesna | 2g |
| 3. | HPBCD | 20g |
| 4. | Disodium hydrogen phosphate | 0.1g |
| 5. | Sodium dihydrogen phosphate | 0.06g |
| 6. | Water | q.s. to 200 ml |

Weighed quantities of disodium hydrogen phosphate and sodium dihydrogen phosphate were dissolved in 160ml of water and a weighed quantity of HPBCD was added and dissolved slowly under stirring.

A weighed quantity of Ifosfamide was gradually added under stirring to the buffered HPBCD solution and mixed for 3 hours.

After 3 hours, a weighed quantity of Mesna was gradually added under stirring to the buffered Ifosfamide solution. The volume was made up to 200 ml with water and filtered through a 0.2µ filter and filled aseptically in sterile glass vials. The glass vials were closed under aseptic conditions with sterile Teflon^{™} coated rubber bungs and sealed using flip off seals.

### Example IV:

| | | |
|---|---|---|
| 1. | Ifosfamide | 10g |
| 2. | Mesna | 2g |
| 3. | HPBCD | 80g |
| 4. | Disodium hydrogen phosphate | 0.1g |
| 5. | Sodium dihydrogen phosphate | 0.06g |
| 6. | Water | q.s. to 200 ml |

The procedure of Example I was repeated using the components in the amounts set forth above.

### Example V:

| | | |
|---|---|---|
| 1. | Ifosfamide | 10g |
| 2. | Mesna | 6g |
| 3. | HPBCD | 20g |
| 4. | Disodium hydrogen phosphate | 0.1g |
| 5. | Sodium dihydrogen phosphate | 0.06g |
| 6. | Water | q.s. to 200 ml |

The procedure of Example I was repeated using the components in the amounts set forth above.

### Example VI:

| | | |
|---|---|---|
| 1. | Ifosfamide | 10g |
| 2. | Mesna | 16g |
| 3. | HPBCD | 20g |
| 4. | Disodium hydrogen phosphate | 0.1g |
| 5. | Sodium dihydrogen phosphate | 0.06g |
| 6. | Water | q.s. to 200 ml |

The procedure of Example I was repeated using the components in the amounts set forth above.

### Example VII:

| | | |
|---|---|---|
| 1. | Ifosfamide | 100g |
| 2. | Mesna | 20g |
| 3. | HPBCD | 10g |
| 4. | Water | q.s. to 200 ml |

A weighed quantity of HPBCD was dissolved in 20 ml of water. Ifosfamide was added gradually to HPBCD solution under stirring and mixing was continued until a clear solution was obtained. Mesna was added gradually under stirring to the resultant Ifosfamide solution and mixed well until the entire quantity of Mesna went into solution. Volume was made up to 200 ml with water.

The composition obtained in this Example was analysed for Ifosfamide content and Mesna content and was found to contain 497.88 mg/ml of Ifosfamide and 98.73 mg/ml of Mesna.

### Example VIII:

| | | |
|---|---|---|
| 1. | Ifosfamide | 100g |
| 2. | Mesna | 60g |
| 3. | HPBCD | 10g |
| 4. | Water | q.s. to 200 ml |

The procedure of Example VII was repeated using the components in the amounts set forth above.

The composition obtained in this Example was analysed for Ifosfamide content and Mesna content and was found to contain 492.02 mg/ml of Ifosfamide and 296.18 mg/ml of Mesna.

### Example IX:

| | | |
|---|---|---|
| 1. | Ifosfamide | 100g |
| 2. | Mesna | 60g |
| 3. | HPBCD | 10g |
| 4. | Disodium hydrogen phosphate | 0.8g |
| 5. | Sodium dihydrogen phosphate | 0.44g |
| 6. | Disodium edetate | 0.01 g |
| 7. | Water | q.s. to 200 ml |

Disodium hydrogen phosphate and Sodium dihydrogen phosphate were dissolved in 20ml of water and then HPBCD was dissolved in the resultant buffer solution. Ifosfamide was then added gradually to HPBCD solution under stirring and mixing was continued until a clear solution was obtained. Disodium edetate was dissolved in minimum quantity of water and was added to Ifosfamide solution. Mesna was added gradually under stirring to the resultant Ifosfamide solution containing edetate and mixed well until the entire quantity of Mesna went into solution. Volume was made up to 200 ml with water.

### Example X:

| | | |
|---|---|---|
| 1. | Ifosfamide | 180g |
| 2. | Mesna | 36g |
| 3. | HPBCD | 10g |
| 4. | Water | q.s. to 200 ml |

The procedure of Example VII was repeated using the components in the amounts set forth above.

### Example XI:

| | | |
|---|---|---|
| 1. | Ifosfamide | 10g |
| 2. | Mesna | 2g |
| 3. | HPBCD | 20g |
| 4. | Disodium hydrogen phosphate | 0.1g |
| 5. | Sodium dihydrogen phosphate | 0.06g |
| 6. | Water | q.s. to 200 ml |

Weighed quantities of disodium hydrogen phosphate and sodium dihydrogen phosphate were dissolved in 160 ml of water and a weighed quantity of HPBCD was added and dissolved slowly under stirring. A weighed quantity of Ifosfamide was gradually added under stirring to buffered HPBCD solution and mixed for 3 hours.

A weighed quantity of Mesna was gradually added under stirring to the above Ifosfamide solution and mixed well.

The volume was made up to 200 ml with water. The product was filtered through a 0.2µ filter and filled aseptically in sterile glass vials. The glass vials were closed under aseptic conditions with sterile Teflon^{™} coated rubber bungs and sealed using flip off seals.

The composition obtained in this Example was analysed for Ifosfamide content and Mesna content and was found to contain 51.2 mg/ml of Ifosfamide and 10 mg/ml of Mesna. The composition had a pH of 7.05.

### Example XII:

The composition obtained in Example XI along with the conventional formulation Holoxan^{TM} were subjected to Haemorrhagic cystitis studies in rats to evaluate the bladder toxicity.

Experimental details are as follows:

| | |
|---|---|
| **Animals used:** | Wistar rats of either sex. |
| Weight range of animals: | 100-150 gm. |
| Number of groups: | 5 |
| Number of animals per group: | 2 |
| Acclimatization: | One week under test conditions, maintained at a temperature of 24 ± 3° and relative humidity of 55% ± 15%. |
| **Test Material:** | Ifosfamide with Mesna Injection. |
| Identity: | Composition of Example XI. |
| Description: | Clear colourless solution |
| Route of administration: | Intravenous |
| **Comparative material:** | Holoxan^{™} |
| Identity: | Ifosfamide injection U.S.P. German Remedies Limited. Lot No.: G 220 Manufacturing Date: October 2001 Expiry Date: September 2003 |
| Description: | Dry powder reconstituted with water for injection to 40 mg/ml |
| Route of administration: | Intravenous |

### Study design

The animals were divided into 5 groups of two animals of each sex and intravenously injected with Ifosfamide formulations as specified in Table I below.

**Table I. Doses of Ifosfamide Formulation.**

| **Group** | **Formulation** | **Dose (mg/kg body weight)** | |
|---|---|---|---|
| **No.** | | **Ifosfamide** | **Mesna** |
| 1 | Holoxan^{™} | 400 | 80 |
| 2 | Holoxan^{™} | 500 | 100 |
| 3 | Example XI | 400 | 80 |
| 4 | Example XI | 500 | 100 |
| 5 | Dextrose Inj. | - | - |

The animals were sacrificed 24 hours after injection. The urinary bladder of all the animals were collected and fixed in 10% formalin for 48 hours. Histopathological slides of the organ were prepared and subjected to microscopic examination.

### Evaluation:

Table 2 represents the grading pattern for haemorrhagic cystitis.

**Table 2 Grading pattern for haemorrhagic cystitis.**

| **Grading** | **Score** |
|---|---|
| Normal | 0 (N) |
| Mild haemorrhagic cystitis | 1 + |
| Moderate haemorrhagic cystitis with or without epithelial atypia | 2 + |
| Severe haemorrhagic cystitis with or without epithelial atypia | 3 + |

### Observations:

Table 3 depicts the evaluation results on haemorrhagic cystitis of the two Ifosfamide formulations

**Table 3 : Evaluations of Ifosfamide formulations for haemorrhagic cystitis.**

| **Animal** | **Formulation** | **Dose (mg/kg)** | | **Score** |
|---|---|---|---|---|
| **No.** | | **Ifosfamide** | **Mesna** | |
| 1. | Holoxan^{™} | 400 | 80 | 1 + |
| 2. | Holoxan^{™} | 400 | 80 | 1 + |
| 3. | Holoxan^{™} | 500 | 100 | 1 + |
| 4. | Holoxan^{™} | 500 | 100 | 2 + |
| 5. | Example XI | 400 | 80 | N* |
| 6. | Example XI | 400 | 80 | N |
| 7. | Example XI | 500 | 100 | N* |
| 8. | Example XI | 500 | 100 | N |
| 9. | Dextrose | - | - | N |
| 10 | Dextrose | - | - | N |

| | | | | |
|---|---|---|---|---|
| * minimal focal submucosal edema & inflammation. No haemorrhage) | | | | |

### Discussion:

Animals treated with Holoxan^{™} showed haemorrhagic cystitis at doses of both 400 mg/kg and 500 mg/kg whereas the composition of Example XI did not show haemorrhagic cystitis.

### Conclusion:

The above findings conclusively proved that the composition of Example XI is less toxic than the conventional formulation Holoxan^{™}.

### Example XIII:

The composition obtained in Example XI was subjected to stability studies. The data is as follows :

| **Storage condition** | **Description** | **Ifosfamide content** (mg/ml) |
|---|---|---|
| Initial | Clear, colourless liquid | 51.2 |
| 2°C-8°C - 3M | Clear, colourless liquid | 50.06 |
| 2°C-8°C - 6M | Clear, colourless liquid | 50.33 |

### Conclusion:

From the above it is evident that Ifosfamide is stable in the composition obtained in Example XI without undergoing any degradation when stored at 2°C - 8°C whereas the conventional formulation on reconstitution is reported to be stable for three to six weeks under refrigeration.

## Claims

1. A process for preparation of a stable aqueous oxazaphosphorine-containing composition comprising an oxazaphosphorine antineoplastic, a 2-mercaptoethanesulphonate and an etherified β-cyclodextrin, said process comprising adding the 2-mercaptoethanesulphonate to an aqueous solution of the oxazaphosphorine antineoplastic and the etherified β-cyclodextrin.

2. A process as claimed in Claim 1, wherein :
the oxazaphosphorine antineoplastic is added to an aqueous solution of the etherified β-cyclodextrin;
the 2-mercaptoethanesulphonate is added to an aqueous solution optionally containing an etherified β-cyclodextrin; and
both the resultant aqueous solutions are mixed together and, optionally, the volume made up with water.

3. A process as claimed in Claim 1, wherein the oxazaphosphorine antineoplastic is added to an aqueous solution of the etherified β-cyclodextrin and subsequently the 2-mercaptoethanesulphonate is directly added to the oxazaphosphorine-containing solution.

4. A process as claimed in any one of the preceding claims, wherein the oxazaphosphorine antineoplastic is of the formula: 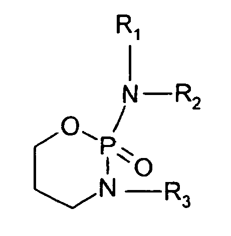 in which at least two of R₁, R₂ and R₃ independently are 2-chloroethyl or 2-methanesulfonyloxyethyl and any remaining R radical is selected from hydrogen, methyl and ethyl.

5. A process as claimed in Claim 4, wherein the oxazaphosphorine antineoplastic is Cyclophosphamide (R₁ = R₂ = chloroethyl & R₃ = hydrogen).

6. A process as claimed in Claim 4, wherein the oxazaphosphorine antineoplastic is Ifosfamide (R₁ = R₃ = chloroethyl & R₂ = hydrogen).

7. A process as claimed in any one of the preceding claims, wherein the 2-mercaptoethanesulphonate is sodium 2-mercaptoethanesulphonate (Mesna).

8. A process as claimed in any one of the preceding claims, wherein the etherified β-cyclodextrin has at least some of the hydroxy groups etherified with hydroxyalkyl groups and, optionally, others etherified with alkyl groups and has a water-solubility of more than 1.8 g/100 ml water.

9. A process as claimed in Claim 8, wherein the hydroxyalkyl groups are hydroxyethyl, dihydroxypropyl or hydroxypropyl groups and the alkyl groups, if present, are methyl or ethyl groups;

10. A process as claimed in any one of the preceding claims, wherein the etherified β-cyclodextrin is hydroxypropyl β-cyclodextrin having a molar substitution per anhydro glucose unit of 0.3 to 0.9.

11. A process as claimed in Claim 10, wherein said molar substitution is 0.5 to 0.8.

12. A process as claimed in any one of the preceding claims, wherein the oxazaphosphorine antineoplastic content of the composition is from 1 mg/ml to 1,000 mg/ml.

13. A process as claimed in any one of the preceding claims, wherein the oxazaphosphorine antineoplastic content of the composition is from 25 mg/ml to 750 mg/ml.

14. A process as claimed in Claim 13, wherein the oxazaphosphorine antineoplastic content of the composition is from 50 mg/ml to 500 mg/ml.

15. A process as claimed in Claim 12, wherein the oxazaphosphorine antineoplastic content of the composition is from 1 mg/ml to 200 mg/ml.

16. A process as claimed in Claim 15, wherein said oxazaphosphorine antineoplastic content is from 10 mg/ml to 100 mg/ml.

17. A process as claimed in Claim 16, wherein said oxazaphosphorine antineoplastic content is from 40 mg/ml to 50 mg/ml.

18. A process as claimed in any one of the preceding claims, wherein the ratio of oxazaphosphorine antineoplastic to 2-mercaptoethanesulphonate is in the range of 20 : 1 to 1 : 2 on a weight basis.

19. A process as claimed in Claim 18, wherein the ratio of oxazaphosphorine antineoplastic to 2-mercaptoethanesulphonate is in the range of 10 : 1 to 1 : 2 on a weight basis.

20. A process as claimed in Claim 19, wherein the ratio of oxazaphosphorine antineoplastic to 2-mercaptoethanesulphonate is in the range of 10 : 1 to 1 : 1 on a weight basis.

21. A process as claimed in any one of the preceding claims, wherein the content of etherified β-cyclodextrin in the composition is from 1% to 60% w/v.

22. A process as claimed in Claim 21, wherein said etherified β-cyclodextrin content is from 2.5% to 40% w/v.

23. A process as claimed in Claim 22, wherein said etherified β-cyclodextrin content is from 5% to 40% w/v.

24. A process as claimed in Claim 23 wherein said etherified β-cyclodextrin content is from 10% to 20% w/v.

25. A process as claimed in Claim 23, wherein said etherified β-cyclodextrin content is from 5% to 20% w/v.

26. A process as claimed in any one of the preceding claims, wherein the resultant aqueous solution containing the oxazaphosphorine antineoplastic, 2-mercaptoethanesulphonate and etherified β-cyclodextrin is sterilized by filtering.

27. A process as claimed in Claim 26, wherein filtrate from the sterilization filter is aseptically filled into a sterile container and the filled container purged with nitrogen and sealed.

28. A stable oxazaphosphorine-containing composition obtainable by a process as claimed in any one of the preceding claims.

29. A stable oxazaphosphorine-containing composition prepared by a process as claimed in any one of the preceding claims.

30. The use of a stable oxazaphosphorine-containing composition as defined in Claim 28 or Claim 29 in the manufacture of a medicament for the treatment of malignant disease.

## Patentansprüche

1. Verfahren zur Herstellung einer stabilen, Oxazaphosphorin enthaltenden Zusammensetzung, umfassend ein Oxazaphosphorinantineoplastik, ein 2-Mercaptoethansulphonat und ein verethertes β-Cyclodextrin, wobei das Verfahren die Zugabe des 2-Merkaptoethansulfonats zu einer wässrigen Lösung des Oxazaphosphorinantineoplastik und des veretherten β-Zyclodextrins umfasst.

2. Verfahren nach Anspruch 1, wobei:
dass Oxazaphosphorinantineoplastik einer wässrigen Lösung des veretherten β-Cyclodextrins zugegeben wird;
das 2-Mercaptoethansulfonat einer wässrigen Lösung zugegeben wird, die optional ein verethertes β-Cyclodextrin enthält; und
beide resultierenden wässrigen Lösungen miteinander vermischt werden und gegebenenfalls das Volumen mit Wasser aufgefüllt wird.

3. Verfahren nach Anspruch 1, wobei das Oxazaphosphorinantineoplastik einer wässrigen Lösung des veretherten β-Cyclodextrins zugegeben wird und nachfolgend das 2-Mercaptoethansulfonat der Oxazaphosphorin enthaltenden Lösung direkt zugegeben wird.

4. Verfahren nach einem der vorstehenden Ansprüche, wobei das Oxazaphosphorinantineoplastik folgende Formel hat: 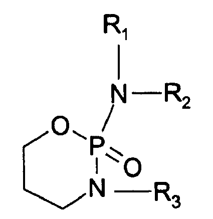 bei der zumindest zwei der Bestandteile R₁, R₂ und R₃ unabhängig voneinander 2-Chloroethyl oder 2-Methansulfonyloxyethyl darstellen und jedes verbleibende R-radikal ausgewählt ist aus Wasserstoff, Methyl und Ethyl.

5. Verfahren nach Anspruch 4, wobei das Oxazaphosphorinantineoplastik Cyclophosphamid (R₁= R₂ = Chloroethyl & R₃ = Wasserstoff) ist.

6. Verfahren nach Anspruch 4, wobei das Oxazaphosphorinantineoplastik Ifosfamid (R₁ = R₃ = Chloroethyl und R₂ = Wasserstoff) ist.

7. Verfahren nach einem der vorstehenden Ansprüche, wobei das 2-Merkaptoethansulfonat Natrium-2-Merkaptoethansulfonat (Mesna) ist.

8. Verfahren nach einem der vorstehenden Ansprüche, wobei das veretherte β-Cyclodextrin mindestens eine der Hydroxygruppen hat, die mit Hydroxyalkylgruppen verethert sind, und gegebenenfalls weitere, die mit Alkylgruppen verethert sind und eine Wasserlöslichkeit von mehr als 1,8 g/100 ml Wasser haben.

9. Verfahren nach Anspruch 8, wobei Hydroxygruppen Hydroxyethyl, Dihydroxypropyl, oder Hydroxypropyl-Gruppen sind und die Alkylgruppen, wenn sie vorliegen, Methyl- oder Ethylgruppen sind.

10. Verfahren nach einem der vorstehenden Ansprüche, wobei das veretherte β-Cyclodextrin ein Hydroxypropyl-β-Cyclodextrin ist, das eine molare Substitution pro Anhydroglukoseeinheit im Bereich von 0,3 bis 0,9 hat.

11. Verfahren nach Anspruch 10, wobei die molare Substitution im Bereich von 0,5 bis 0,8 ist.

12. Verfahren nach einem der vorstehenden Ansprüche, wobei der Oxazaphosphorinantineoplastik-Gehalt der Zusammensetzung im Bereich von 1 mg/ml bis 1000 mg/ml liegt.

13. Verfahren nach einem der vorstehenden Ansprüche, wobei der Oxazaphosphorinantineoplastik-Gehalt der Zusammensetzung im Bereich von 25 mg/ml bis 750 mg/ml liegt.

14. Verfahren nach Anspruch 13, wobei der Oxazaphosphorinantineoplastik-Gehalt der Zusammensetzung im Bereich von 50 mg/ml bis 500 mg/ml liegt.

15. Verfahren nach Anspruch 12, wobei der Oxazaphosphorinantineoplastik-Gehalt der Zusammensetzung im Bereich von 1 mg/ml bis 200 mg/ml liegt.

16. Verfahren nach Anspruch 15, wobei der Oxazaphosphorinantineoplastik-Gehalt im Bereich von 10 mg/ml bis 100 mg/ml liegt.

17. Verfahren nach Anspruch 16, wobei der Oxazaphosphorinantineoplastik-Gehalt im Bereich von 40 mg/ml bis 50 mg/ml liegt.

18. Verfahren nach einem der vorstehenden Ansprüche, wobei das Verhältnis von Oxazaphosphorinantineoplastik zu 2-Merkaptoethansulfonat in dem Bereich von 20:1 bis 1:2 auf Gewichtsbasis liegt.

19. Verfahren nach Anspruche 18, wobei das Verhältnis von Oxazaphosphorinantineoplastik zu 2-Merkaptoethansulfonat in dem Bereich von 10:1 bis 1:2 auf Gewichtsbasis liegt.

20. Verfahren nach Anspruch 19, wobei das Verhältnis von Oxazaphosphorinantineoplastik zu 2-Merkaptoethansulfonat in Bereich von 10:1 bis 1:1 auf Gewichtsbasis liegt.

21. Verfahren nach einem der vorstehenden Ansprüche, wobei der Gehalt an verethertem β-Cyclodextrin in der Zusammensetzung im Bereich von 1% bis 60% w/v ist.

22. Verfahren nach Anspruch 21, wobei der Gehalt an verethertem β-Cyclodextrin im Bereich von 2,5% bis 40% w/v ist.

23. Verfahren nach Anspruch 22, wobei der Gehalt an verethertem β-Cyclodextrin im Bereich von 5% bis 40% w/v ist.

24. Verfahren nach Anspruch 23, wobei der Gehalt an verethertem β-Cyclodextrin im Bereich von 10% bis 20% w/v ist.

25. Verfahren nach Anspruch 23, wobei der Gehalt an verethertem β-Cyclodextrin im Bereich von 5% bis 20% w/v ist.

26. Verfahren nach einem der vorstehenden Ansprüche, wobei die resultierende wässrige Lösung, die das Oxazaphosphorinantineoplastik, das 2-merkaptoethansulfunat und das veretherte β-Cyclodextrin enthält, durch Filtrieren sterilisiert wird.

27. Verfahren nach Anspruch 26, wobei ein Filtrat aus dem Sterilisationsfilter keimfrei in einen sterilen Behälter gefüllt wird und der befüllte Behälter mit Stickstoff geklärt und versiegelt wird.

28. Stabile Oxazaphosphorin enthaltende Zusammensetzung, durch ein Verfahren nach einem der vorstehenden Ansprüche erhältlich ist.

29. Stabile Oxazaphosphorin enthaltende Zusammensetzung, die durch ein Verfahren nach einem der vorstehenden Ansprüche hergestellt ist.

30. Verwendung einer stabilen Oxazaphosphorin enthaltenden Zusammensetzung nach Anspruch 28 oder 29 bei der Herstellung eines Medikaments zur Behandlung einer bösartigen Krankheit.

## Revendications

1. Procédé de préparation d'une composition aqueuse stable contenant une oxazaphosphorine comprenant un antinéoplasique à base d'oxazaphosphorine, un 2-mercaptoéthanesulfonate et une β-cyclodextrine éthérifiée, ledit procédé comprenant l'ajout du 2-mercaptoéthanesulfonate à une solution aqueuse de l'antinéoplasique à base d'oxazaphosphorine et de la β-cyclodextrine éthérifiée.

2. Procédé selon la revendication 1, dans lequel :
l'antinéoplasique à base d'oxazaphosphorine est ajouté à une solution aqueuse de la β-cyclodextrine éthérifiée ;
le 2-mercaptoéthanesulfonate est ajouté à une solution aqueuse contenant éventuellement une β-cyclodextrine éthérifiée ; et
les deux solutions aqueuses résultantes sont mélangées ensemble et, éventuellement, le volume est complété avec de l'eau.

3. Procédé selon la revendication 1, dans lequel l'antinéoplasique à base d'oxazaphosphorine est ajouté à une solution aqueuse de la β-cyclodextrine éthérifiée, et ultérieurement, le 2-mercaptoéthanesulfonate est directement ajouté à la solution contenant l'oxazaphosphorine.

4. Procédé selon l'une quelconque des revendications précédentes, dans lequel l'antinéoplasique à base d'oxazaphosphorine répond à la formule : 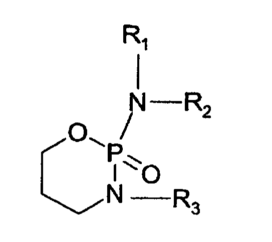 dans laquelle au moins deux des radicaux R₁, R₂ et R₃ représentent indépendamment un groupe 2-chloroéthyle ou 2-méthanesulfonyloxyéthyle et tout radical R restant est choisi parmi un atome d'hydrogène, un groupe méthyle et un groupe éthyle.

5. Procédé selon la revendication 4, dans lequel l'antinéoplasique à base d'oxazaphosphorine est le Cyclophosphamide (R₁ = R₂ = un groupe chloroéthyle et R₃ = un atome d'hydrogène).

6. Procédé selon la revendication 4, dans lequel l'antinéoplasique à base d'oxazaphosphorine est l' Ifosfamide (R₁ = R₃ = un groupe chloroéthyle et R₂ = un atome d'hydrogène).

7. Procédé selon l'une quelconque des revendications précédentes, dans lequel le 2-mercaptoéthanesulfonate est le 2-mercaptoéthanesulfonate de sodium (Mesna).

8. Procédé selon l'une quelconque des revendications précédentes, dans lequel la β-cyclodextrine éthérifiée a au moins certains des groupes hydroxy éthérifiés avec des groupes hydroxyalkyle et, éventuellement, d'autres éthérifiés avec des groupes alkyle et a une hydrosolubilité supérieure à 1,8 g/100 ml d'eau.

9. Procédé selon la revendication 8, dans lequel les groupes hydroxyalkyle sont des groupes hydroxyéthyle, dihydroxypropyle ou hydroxypropyle et les groupes alkyle, s'ils sont présents, sont des groupes méthyle ou éthyle.

10. Procédé selon l'une quelconque des revendications précédentes, dans lequel la β-cyclodextrine éthérifiée est une hydroxypropyl-β-cyclodextrine ayant une substitution molaire par motif d'anhydro-glucose de 0,3 à 0,9.

11. Procédé selon la revendication 10, dans lequel ladite substitution molaire est de 0,5 à 0,8.

12. Procédé selon l'une quelconque des revendications précédentes, dans lequel la teneur en antinéoplasique à base d'oxazaphosphorine de la composition est de 1 mg/ml à 1 000 mg/ml.

13. Procédé selon l'une quelconque des revendications précédentes, dans lequel la teneur en antinéoplasique à base d'oxazaphosphorine de la composition est de 25 mg/ml à 750 mg/ml.

14. Procédé selon la revendication 13, dans lequel la teneur en antinéoplasique à base d'oxazaphosphorine de la composition est de 50 mg/ml à 500 mg/ml.

15. Procédé selon la revendication 12, dans lequel la teneur en antinéoplasique à base d'oxazaphosphorine de la composition est de 1 mg/ml à 200 mg/ml.

16. Procédé selon la revendication 15, dans lequel ladite teneur en antinéoplasique à base d'oxazaphosphorine est de 10 mg/ml à 100 mg/ml.

17. Procédé selon la revendication 16, dans lequel ladite teneur en antinéoplasique à base d'oxazaphosphorine est de 40 mg/ml à 50 mg/ml.

18. Procédé selon l'une quelconque des revendications précédentes, dans lequel le rapport de l'antinéoplasique à base d'oxazaphosphorine au 2-mercaptoéthanesulfonate est dans la gamme de 20/1 à 1/2 sur la base du poids.

19. Procédé selon la revendication 18, dans lequel le rapport de l'antinéoplasique à base d'oxazaphosphorine au 2-mercaptoéthanesulfonate est dans la gamme de 10/1 à 1/2 sur la base du poids.

20. Procédé selon la revendication 19, dans lequel le rapport de l'antinéoplasique à base d'oxazaphosphorine au 2-mercaptoéthanesulfonate est dans la gamme de 10/1 à 1/1 sur la base du poids.

21. Procédé selon l'une quelconque des revendications précédentes, dans lequel la teneur en β-cyclodextrine éthérifiée dans la composition est de 1 % à 60 % en poids par volume.

22. Procédé selon la revendication 21, dans lequel ladite teneur en β-cyclodextrine éthérifiée est de 2,5 % à 40 % en poids par volume.

23. Procédé selon la revendication 22, dans lequel ladite teneur en β-cyclodextrine éthérifiée est de 5 % à 40 % en poids par volume.

24. Procédé selon la revendication 23, dans lequel ladite teneur en β-cyclodextrine éthérifiée est de 10 % à 20 % en poids par volume.

25. Procédé selon la revendication 23, dans lequel ladite teneur en β-cyclodextrine éthérifiée est de 5 % à 20 % en poids par volume.

26. Procédé selon l'une quelconque des revendications précédentes, dans lequel la solution aqueuse résultante contenant l'antinéoplasique à base d'oxazaphosphorine, le 2-mercaptoéthanesulfonate et la β-cyclodextrine éthérifiée est stérilisée par filtration.

27. Procédé selon la revendication 26, dans lequel le filtrat issu du filtre de stérilisation est versé de manière aseptique dans un récipient stérile et le récipient rempli est purgé avec de l'azote et est scellé.

28. Composition stable contenant une oxazaphosphorine que l'on peut obtenir au moyen d'un procédé selon l'une quelconque des revendications précédentes.

29. Composition stable contenant une oxazaphosphorine préparée au moyen d'un procédé selon l'une quelconque des revendications précédentes.

30. Utilisation d'une composition stable contenant une oxazaphosphorine selon la revendication 28 ou la revendication 29 dans la fabrication d'un médicament destiné au traitement d'une maladie maligne.
